# EUROPEAN PATENT APPLICATION

(11) **EP 2 881 045 A1**
(43) Date of publication of application: **10.06.2015**
(21) Application number: 14196148.2
(22) Date of filing: 03.12.2014
(51) Int. Cl.: A61B 17/072, A61B 17/00, A61B 17/29

(54) **Adapter direct drive push button retention mechanism**

(30) Priority: 04.12.2013 US 201361911781 P; 21.11.2014 US 201414549643
(71) Applicant: Covidien LP, Mansfield, MA 02048 (US)
(72) Inventor: Richard, Paul D., Shelton, Connecticut 06484 (US); Zergiebel, Earl M., Guilford, Connecticut 06430 (US); Chowaniec, David M., Rocky Hill, Connecticut 06067 (US); Williams, Ryan V., New Hartford, Connecticut 06057 (US); Subramanian, Anand, Stamford, Connecticut 06902 (US)
(74) Representative: Soames, Candida Jane

(57) **Abstract**

A surgical device adapter for coupling an end effector to a handle assembly is disclosed. The surgical device adapter includes: a housing including a proximal end couplable to a distal end of a handle assembly; and a drive coupling assembly selectively couplable to the handle assembly, the drive coupling assembly including a lock assembly having a pair of spring-loaded, opposing buttons movable from a clamped configuration in which the pair of opposing buttons are engaged to the distal end of the handle assembly to an unclamped configuration in which the pair of opposing buttons are disengaged from the distal end of the handle assembly.

## Description

### CROSS-REFERENCE TO RELATED APPLICATIONS

This application claims the benefit of and priority to U.S. Provisional Patent Application No. 61/911,781, filed December 4, 2013, the entire disclosure of which is incorporated by reference herein.

### BACKGROUND

### 1. Technical Field

The present disclosure relates to surgical apparatuses, devices and/or systems for performing endoscopic surgical procedures and methods of use thereof. More specifically, the present disclosure relates to electromechanical hand-held surgical apparatuses, adapters, devices and/or systems configured for use with removable disposable loading units and/or single use loading units for clamping, cutting and/or stapling tissue.

### 2. Background of Related Art

Currently there are various drive systems for operating and/or manipulating electromechanical surgical devices. In many instances the electromechanical surgical devices include a reusable handle assembly, and disposable or single-use loading units. The loading units are selectively connected to the handle assembly prior to use and then disconnected from the handle assembly following use in order to be disposed of or in some instances sterilized for re-use.

Many of the existing end effectors for use with many of the existing surgical devices and/or handle assemblies are driven by a linear force. For example, end effectors for performing endo-gastrointestinal anastomosis procedures, end-to-end anastomosis procedures and transverse anastomosis procedures, each typically require a linear driving force in order to be operated. As such, these end effectors are not compatible with surgical devices and/or handle assemblies that use rotary motion to deliver power or the like.

In order to make the linear driven end effectors compatible with surgical devices and/or handle assemblies that use a rotary motion to deliver power, a need exists for adapters and/or adapter assemblies to interface between and interconnect the linear driven end effectors with the rotary driven surgical devices and/or handle assemblies. There is also a need for adapters that include manual retraction, connection, and locking and release mechanisms for coupling to the surgical devices.

### SUMMARY

Further details and aspects of exemplary embodiments of the present invention are described in more detail below with reference to the appended Figures.

According to one embodiment of the present disclosure, a surgical device adapter for coupling an end effector to a handle assembly is disclosed. The surgical device adapter includes: a housing including a proximal end couplable to a distal end of a handle assembly; and a drive coupling assembly selectively couplable to the handle assembly, the drive coupling assembly including a lock assembly having a pair of spring-loaded, opposing buttons movable from a clamped configuration in which the pair of opposing buttons are engaged to the distal end of the handle assembly to an unclamped configuration in which the pair of opposing buttons are disengaged from the distal end of the handle assembly.

According to one aspect of the present disclosure, each of the buttons includes a tab projecting toward one another, whereby each tab is configured and dimensioned to interface with a circumferential slot disposed about the distal end of the handle assembly. Each of the buttons may also include an actuation portion disposed at an opposite end of the tab.

According to another aspect of the present disclosure, each of the buttons is movable in opposing directions between the clamped configuration and the unclamped configuration. The buttons may be biased in opposite directions away from one another.

According to another embodiment of the present disclosure, a surgical device adapter for coupling an end effector to a handle assembly is disclosed. The surgical device adapter includes: a housing including a proximal end couplable to a distal end of a handle assembly; and a drive coupling assembly selectively couplable to the handle assembly, the drive coupling assembly including a lock assembly having one or more spring-loaded button movable from a clamped configuration in which the at least one button is engaged to the distal end of the handle assembly to an unclamped configuration in which the at least one button is disengaged from the distal end of the handle assembly.

According to one aspect of the present disclosure, the button includes a tab configured and dimensioned to interface with a circumferential slot disposed about the distal end of the handle assembly. The button may include an actuation portion disposed at an opposite end of the tab.

According to another aspect of the present disclosure, the surgical device adapter further includes an opposing, spring-loaded button movable from a clamped configuration in which the buttons are engaged to the distal end of the handle assembly to an unclamped configuration in which the buttons are disengaged from the distal end of the handle assembly. Each of the buttons may be movable in opposing directions between the clamped configuration and the unclamped configuration. The buttons may be biased in opposite directions.

According a further aspect of the present disclosure, the drive coupling assembly includes: a recess defined therein configured and dimensioned to fit about the distal end of the handle assembly; and a pair of lumens transverse to a longitudinal axis defined by the surgical device adapter and at least partially extending through the recess. The button may include a pair of posts disposed within the lumens. Each of the posts may include a depression configured and dimensioned to release the distal end of the handle assembly in the unclamped configuration.

According to a further embodiment of the present disclosure, a surgical device is disclosed. The surgical device includes an end effector comprising a first jaw and a second jaw moveable relative to the first jaw; a handle assembly; and an adapter assembly removably coupled to a proximal end of the end effector and a distal end of the handle assembly. The adapter assembly includes: a housing including a proximal end couplable to the distal end of the handle assembly; and a drive coupling assembly selectively couplable to the handle assembly, the drive coupling assembly including a lock assembly having at least one spring-loaded button movable from a clamped configuration in which the at least one button is engaged to the distal end of the handle assembly to an unclamped configuration in which the at least one button is disengaged from the distal end of the handle assembly.

According to one aspect of the present disclosure, the button includes a tab configured and dimensioned to interface with a circumferential slot disposed about the distal end of the handle assembly. The button may include an actuation portion disposed at an opposite end of the tab.

According to another aspect of the present disclosure, the adapter assembly further includes an opposing, spring-loaded button movable from a clamped configuration in which the buttons are engaged to the distal end of the handle assembly to an unclamped configuration in which the buttons are disengaged from the distal end of the handle assembly. Each of the buttons may be movable in opposing directions between the clamped configuration and the unclamped configuration. The buttons may be biased in opposite directions.

### BRIEF DESCRIPTION OF THE DRAWINGS

Embodiments of the present disclosure are described herein with reference to the accompanying drawings, wherein:
Fig. 1 is a perspective view of an electromechanical surgical system including a surgical instrument, an end effector and an adapter assembly according to the present disclosure;
Fig. 2 is a perspective, front partial view of the surgical instrument of Fig. 1, according to the present disclosure;
Fig. 3 is a perspective, rear view of the adapter assembly of Fig. 1, according to the present disclosure;
Fig. 4 is a further, perspective rear view of the adapter assembly of Fig. 1, according to the present disclosure;
Fig. 5 is a perspective view of a drive coupling assembly of the adapter assembly of Fig. 1 shown decoupled from the surgical instrument, according to one embodiment of the present disclosure;
Fig. 6 is a perspective view of the drive coupling assembly of Fig. 5 shown coupled to the surgical instrument, according to the present disclosure;
Fig. 7 is a cross-sectional, side view of the drive coupling assembly of Fig. 5 shown coupled to the surgical instrument, taken along section line 7-7 of Fig. 6, according to the present disclosure;
Fig. 8 is a perspective view of a lock assembly of the drive coupling assembly of Fig. 5, according to the present disclosure;
Fig. 9 is a perspective view of a drive coupling assembly of the adapter assembly of Fig. 1, according to another embodiment of the present disclosure; and
Fig. 10 is a perspective view of a lock button of the drive coupling assembly of Fig. 9, according to the present disclosure.

### DETAILED DESCRIPTION OF EMBODIMENTS

Embodiments of the presently disclosed electromechanical surgical system, apparatus and/or device are described in detail with reference to the drawings, in which like reference numerals designate identical or corresponding elements in each of the several views. As used herein the term "distal" refers to that portion of the electromechanical surgical system, apparatus and/or device, or component thereof, that are farther from the user, while the term "proximal" refers to that portion of the electromechanical surgical system, apparatus and/or device, or component thereof, that are closer to the user. The terms "left" and "right" refer to that portion of the electromechanical surgical system, apparatus and/or device, or component thereof, that are on the left and right sides, respectively, from the perspective of the user facing the distal end of the electromechanical surgical system, apparatus and/or device from the proximal end while the surgical system, apparatus and/or device is oriented in non-rotational (e.g., home) configuration.

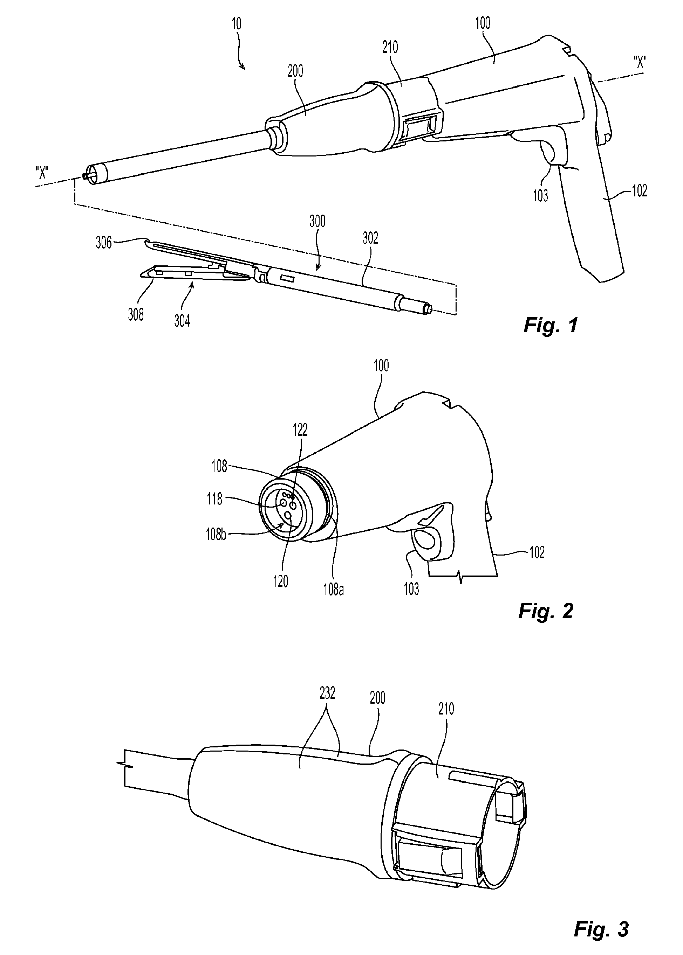

Referring initially to Figs. 1-3, an electromechanical, hand-held, powered surgical system, in accordance with an embodiment of the present disclosure is shown and generally designated 10. Electromechanical surgical system 10 includes a surgical apparatus or device in the form of an electromechanical, hand-held, powered surgical instrument 100 that is configured for selective attachment thereto of a plurality of different end effectors 300, via an adapter assembly 200 (e.g., elongated body). The end effector 300 and the adapter assembly 200 are configured for actuation and manipulation by the electromechanical, hand-held, powered surgical instrument 100. In particular, the surgical instrument 100, the adapter assembly 200, and the end effector 300 are separable from each other such that the surgical instrument 100 is configured for selective connection with adapter assembly 200, and, in turn, adapter assembly 200 is configured for selective connection with any one of a plurality of different end effectors 300.

Reference may be made to International Application No. PCT/US2008/077249, filed September 22, 2008 (Inter. Pub. No. WO 2009/039506) and U.S. Patent Application Publication No. 2011/0121049, the entire contents of all of which are incorporated herein by reference, for a detailed description of the construction and operation of exemplary electromechanical, hand-held, powered surgical instrument 100.

As illustrated in Figs. 1 and 2, surgical instrument 100 includes a handle housing 102 including one or more controllers, a power source, and a drive mechanism having one or more motors, gear selector boxes, gearing mechanisms, and the like. The housing 102 also supports a control assembly 103. Control assembly 103 may include one or more finger-actuated control buttons, rocker devices, joystick or other directional controls, whose input is transferred to the drive mechanism to actuation the adapter assembly 200 and the end effector 300.

In particular, drive mechanism is configured to drive shafts and/or gear components in order to selectively move a tool assembly 304 of end effector 300 relative to proximal body portion 302 of end effector 300, to rotate end effector 300 about a longitudinal axis "X-X" (Fig. 1) defined by the adapter assembly 200 relative to handle housing 102, to move an anvil assembly 306 relative to a cartridge assembly 308 of end effector 300, and/or to fire a stapling and cutting cartridge within cartridge assembly 308 of end effector 300.

With continued reference to Fig. 2, the housing 102 defines a nose or connecting portion 108 configured to accept a corresponding drive coupling assembly 210 of adapter assembly 200. The connecting portion 108 of surgical instrument 100 has a cylindrical recess 108b that receives a connector assembly 240 of the drive coupling assembly 210 when adapter assembly 200 is mated to surgical instrument 100. Connecting portion 108 houses one or more rotatable drive connectors that interface with corresponding rotatable connector sleeves of the adapter assembly 200, as described in further detail below. The surgical instrument 100 includes rotatable drive connector 118, 120, 122 disposed within the connecting portion 108 that are actuated by the drive mechanism thereof (not shown).

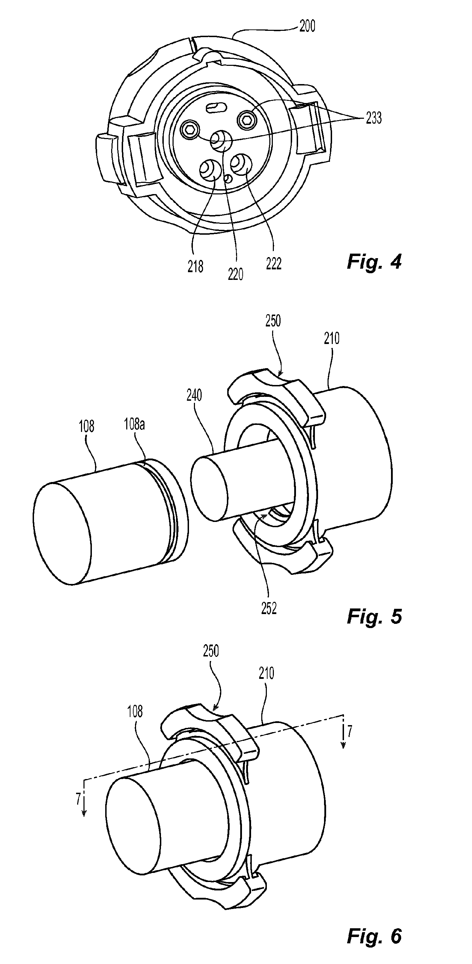

With reference to Figs. 2 and 4, when adapter assembly 200 is mated to surgical instrument 100, each of rotatable drive connectors 118, 120, 122 of surgical instrument 100 couples with a corresponding rotatable connector sleeve 218, 220, 222 of adapter assembly 200. In this regard, the interface between corresponding first drive connector 118 and first connector sleeve 218, the interface between corresponding second drive connector 120 and second connector sleeve 220, and the interface between corresponding third drive connector 122 and third connector sleeve 222 are keyed such that rotation of each of drive connectors 118, 120, 122 of surgical instrument 100 causes a corresponding rotation of the corresponding connector sleeve 218, 220, 222 of adapter assembly 200.

The mating of drive connectors 118, 120, 122 of surgical instrument 100 with connector sleeves 218, 220, 222 of adapter assembly 200 allows rotational forces to be independently transmitted via each of the three respective connector interfaces. The drive connectors 118, 120, 122 of surgical instrument 100 are configured to be independently rotated by drive mechanism.

Since each of drive connectors 118, 120, 122 of surgical instrument 100 has a keyed and/or substantially non-rotatable interface with respective connector sleeves 218, 220, 222 of adapter assembly 200, when adapter assembly 200 is coupled to surgical instrument 100, rotational force(s) are selectively transferred from drive mechanism of surgical instrument 100 to adapter assembly 200.

The selective rotation of drive connector(s) 118, 120 and/or 122 of surgical instrument 100 allows surgical instrument 100 to selectively actuate different functions of end effector 300. As described in greater detail below, selective and independent rotation of first drive connector 118 of surgical instrument 100 corresponds to the selective and independent opening and closing of tool assembly 304 of end effector 300, and driving of a stapling/cutting component of tool assembly 304 of end effector 300. Also, the selective and independent rotation of second drive connector 120 of surgical instrument 100 corresponds to the selective and independent articulation of tool assembly 304 of end effector 300 about an articulation axis that is transverse to longitudinal axis "X-X" (Fig. 1). In particular, the end effector 300 defines a second longitudinal axis and is movable from a first position in which the second longitudinal axis is substantially aligned with the first longitudinal axis "X-X" to at least a second position in which the second longitudinal axis is disposed at a non-zero angle with respect to the first longitudinal axis "X-X." Additionally, the selective and independent rotation of third drive connector 122 of surgical instrument 100 corresponds to the selective and independent rotation of end effector 300 about longitudinal axis "X-X" (Fig. 1) relative to handle housing 102 of surgical instrument 100.

In embodiments, adapter assembly 200 may include first, second, and third drive transmitting/converting assemblies (not shown). Reference may be made to a commonly-owned U.S. Patent Application No. 13/875,571, entitled, "Hand Held Surgical Handle Assembly, Surgical Adapters For Use Between Surgical Handle Assembly and Surgical End Effectors, and Methods Of Use," which describes construction and operation of exemplary first, second, and third transmitting/converting assemblies, the entire contents of which are incorporated by reference herein.

With reference to Figs. 3 and 4, the adapter assembly 200 also includes an adapter housing 232 coupled to the coupling assembly 210 and enclosing a drive mechanism (not shown). The adapter assembly 200 also includes the connector assembly 240 (Fig. 5) disposed within the coupling assembly 210. The connector assembly 240 is coupled to the adapter housing 232 via fasteners 233 (Fig. 4). The connector assembly 240 is configured and dimensioned to be inserted into the cylindrical recess 108b of the connector portion 108.

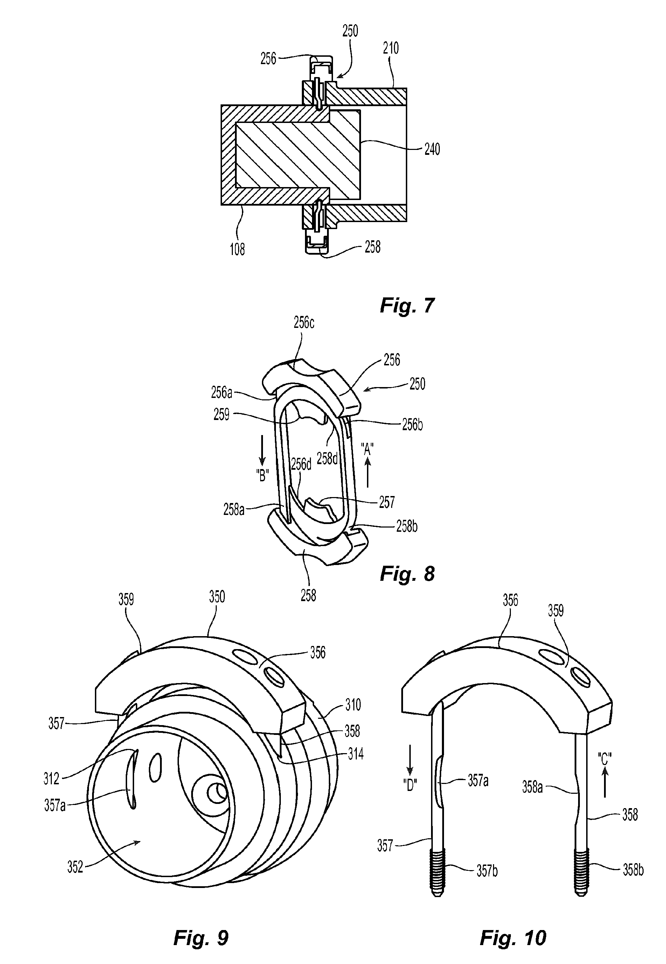

With reference to Figs. 5-8, the drive coupling assembly 210 includes a lock assembly 250 configured to clamp about an outer circumferential slot 108a of the connecting portion 108. The drive coupling assembly 210 includes a recess 252 that receives the connecting portion 108 of the surgical instrument 100 when adapter assembly 200 is mated to surgical instrument 100. The lock assembly 250 is configured to engage the slot 108a to couple the adapter assembly 200 to the surgical instrument 100 once they are mated, as shown in Figs. 6 and 7.

With continued reference to Figs. 5-8, the lock assembly 250 includes a pair of opposing, spring-loaded buttons 256 and 258. Each of the buttons 256, 258 has a substantially annular shape having a pair of opposing, longitudinal chamfered sides 256a, 256b, and 258a, 258b, respectively. Each of the buttons 256, 258 also includes an actuation portion 256c and 258c, respectively, and an engagement portion 256d and 258d, respectively. In embodiments, each of the actuation portions 256c, 258c may include a depression (e.g., curvature) or a textured surface to provide for sufficient grip by the user. Each of the engagement portions 256d, 258d includes a tab 257 and 259 configured to engage the circumferential slot 108a of the connecting portion 108. The lock assembly 250 also includes one or more springs (not shown) coupled to each of the buttons 256, 258, thereby biasing the buttons 256, 258 in directions "A" and "B," respectively, which urges the tabs 257, 259 inwardly to engage the circumferential slot 108a of the connecting portion 108.

The buttons 256, 258 are movable along a plane transverse to the longitudinal axis "X-X" in opposite directions "A" and "B." Disengagement of the tabs 257, 259, from slot 106a is accomplished by pressing on the respective opposed engagement portion 256d, 258d of the buttons 256, 258 in directions "B" and "A," respectively (e.g., opposite direction of the biasing forces). In so doing the tabs 257, 259 are forced radially outward, allowing for insertion or removal of the adapter assembly 200 from the surgical instrument 100.

Figs. 6 and 7 show the buttons 256, 258 coupling the connecting portion 108 of the surgical instrument 100 to the drive coupling assembly 210. Prior to insertion or removal of surgical instrument 100 and drive coupling assembly 210 with/from one another, the buttons 256, 258 are actuated by pressing on the actuation portion 256c, 258c to move the tabs 257, 259 of the respective opposed engagement portion 256d, 258d of the buttons 256, 258 out of the recess 252, thereby allowing for the insertion or removal of the connecting portion 108 into/from the drive coupling assembly 210. During insertion, once the connecting portion 108 is disposed within the drive coupling assembly 210, the buttons 256, 258 are released and are drawn into engagement with the connection portion 108, namely, the tabs 257, 259 of the engagement portions 256d, 258d engage the circumferential slot 108a of the connecting portion 108, thereby coupling the adapter assembly 200 to the surgical instrument 100.

Figs. 9 and 10 show another embodiment of a drive coupling 310, which includes a lock assembly 350 configured to clamp about a circumferential slot 108a of the connecting portion 108 of the surgical instrument 100. The drive coupling assembly 310 includes a recess 352 that receives the connecting portion 108 of the surgical instrument 100 when adapter assembly 200 is mated to surgical instrument 100. The lock assembly 350 is configured to engage the slot 108a to secure the adapter assembly 200 to the surgical instrument 100 once they are mated.

The lock assembly 350 includes a spring-loaded button 356 including a pair of posts 357, 358, which are coupled to an actuation portion 359. The posts are 357, 358 are disposed within lumens 312, 314, respectively, of the drive coupling 310. The lumens 312, 314 are transverse to a longitudinal axis "X-X" and extend at least partially through the recess 352 of the drive coupling 310, such that the posts 357, 358 are exposed within the recess 352 or project radially inward from recess 352.

Each of the posts 357, 358 includes an arcuate depression 357a, 358a, respectively, which is oriented towards one another. Each post 357, 358 also includes a spring 357b, 358b, which engage the posts 357, 358, respectively, tending to bias the button 356 in direction "C." In embodiments, the actuation portion 356 may include a depression (e.g., curvature) or a textured surface to provide for sufficient grip by the user.

The button 356 is movable along a plane transverse to the longitudinal axis "X-X." As the button 356 is pushed radially outward by the springs 357b, 358b in the direction "C," lower portion of the posts 357, 358 (e.g., non-arcuate portion) is disposed within or projects into the recess 352, thereby engaging the circumferential slot 108a of the connecting portion 108 of the surgical instrument 100. Disengagement of the posts 357, 358 is accomplished by pressing on the actuation portion 359 of the button 356 in directions "D" (e.g., opposite direction of the biasing force), which forces the posts 357, 358 downwardly. This movement aligns the arcuate depressions 357a, 358a with the recess 352, allowing for insertion or removal of the adapter assembly 200 from the surgical instrument 100.

Prior to insertion or removal, the button 356 is actuated by pressing on the button 356, which moves the arcuate depressions 357a, 358a of the posts 357, 358 into the recess 352 (e.g., into alignment therewith) allowing for insertion or removal of the connecting portion 108 into the drive coupling assembly 310. Once the connecting portion 108 is disposed within the drive coupling assembly 310, the button 356 is released and is pushed into engagement with the connection portion 108 by the springs 357b, 358b, specifically, the posts 357, 358 engage the circumferential slot 108a of the connecting portion 108, thereby coupling the adapter assembly 200 to the surgical instrument 100.

It will be understood that various modifications may be made to the embodiments disclosed herein. For example, the instrument 100 need not apply staples but rather may apply two part fasteners as is known in the art. Further, the length of the linear row of staples or fasteners may be modified to meet the requirements of a particular surgical procedure. Thus, the length of a single stroke of the actuation shaft and/or the length of the linear row of staples and/or fasteners within a disposable loading unit may be varied accordingly. Therefore, the above description should not be construed as limiting, but merely as exemplifications of preferred embodiments. Those skilled in the art will envision other modifications within the scope and spirit of the claims appended thereto.

The invention may be described by reference to the following numbered paragraphs:-
1. A surgical device adapter for coupling an end effector to a handle assembly, the surgical device adapter comprising:
   a housing including a proximal end couplable to a distal end of a handle assembly; and
   a drive coupling assembly adapted to be selectively couplable to a handle assembly, the drive coupling assembly including a lock assembly having a pair of spring-loaded, opposing buttons movable from a clamped configuration in which the pair of opposing buttons are engaged to a distal end of a handle assembly to an unclamped configuration in which the pair of opposing buttons are disengaged from a distal end of a handle assembly.
2. The surgical device adapter according to paragraph 1, wherein each of the buttons includes a tab projecting toward one another, whereby each tab is configured and dimensioned to interface with a circumferential slot disposed about a distal end of a handle assembly.
3. The surgical device adapter according to paragraph 2, wherein each of the buttons includes an actuation portion disposed at an opposite end of the tab.
4. The surgical device adapter according to paragraph 1, wherein each of the buttons is movable in opposing directions between the clamped configuration and the unclamped configuration.
5. The surgical device adapter according to paragraph 1, wherein the buttons are biased in opposite directions away from one another.
6. A surgical device adapter for coupling an end effector to a handle assembly, the surgical device adapter comprising:
   a housing including a proximal end couplable to a distal end of a handle assembly; and
   a drive coupling assembly adapted to be selectively couplable to a handle assembly, the drive coupling assembly including a lock assembly having at least one spring-loaded button movable from a clamped configuration in which the at least one button is engaged to a distal end of a handle assembly to an unclamped configuration in which the at least one button is disengaged from a distal end of a handle assembly.
7. The surgical device adapter according to paragraph 6, wherein the at least one button includes a tab configured and dimensioned to interface with a circumferential slot disposed about a distal end of a handle assembly.
8. The surgical device adapter according to paragraph 6, further comprising an opposing, spring-loaded button movable from a clamped configuration in which the buttons are engaged to the distal end of a handle assembly to an unclamped configuration in which the buttons are disengaged from a distal end of a handle assembly.
9. The surgical device adapter according to paragraph 8, wherein each of the buttons is movable in opposing directions between the clamped configuration and the unclamped configuration.
10. The surgical device adapter according to paragraph 8, wherein the buttons are biased in opposite directions.
11. The surgical device adapter according to paragraph 6, wherein the drive coupling assembly includes:
   a recess defined therein, the recess being configured and dimensioned to receive a distal end of a handle assembly; and
   a pair of lumens transverse to a longitudinal axis defined by the surgical device adapter and at least partially extending through the recess.
12. The surgical device adapter according to paragraph 11, wherein the at least one button includes a pair of posts disposed within the lumens.
13. The surgical device adapter according to paragraph 11, wherein each of the posts includes a depression configured and dimensioned to release a distal end of a handle assembly in the unclamped configuration.
14. A surgical device comprising:
   an end effector comprising a first jaw and a second jaw moveable relative to the first jaw;
   a handle assembly; and
   an adapter assembly removably coupled to a proximal end of the end effector and a distal end of the handle assembly, the adapter assembly comprising:
      a housing including a proximal end couplable to the distal end of the handle assembly; and
      a drive coupling assembly selectively couplable to the handle assembly, the drive coupling assembly including a lock assembly having at least one spring-loaded button movable from a clamped configuration in which the at least one button is engaged to the distal end of the handle assembly to an unclamped configuration in which the at least one button is disengaged from the distal end of the handle assembly.
15. The surgical device according to paragraph 14, wherein the at least one button includes a tab configured and dimensioned to interface with a circumferential slot disposed about the distal end of the handle assembly.
16. The surgical device according to paragraph 14, wherein the adapter assembly further comprises an opposing, spring-loaded button movable from a clamped configuration in which the buttons are engaged to the distal end of the handle assembly to an unclamped configuration in which the buttons are disengaged from the distal end of the handle assembly.
17. The surgical device according to paragraph 16, wherein each of the buttons is movable in opposing directions between the clamped configuration and the unclamped configuration.
18. The surgical device according to paragraph 17, wherein the buttons are biased in opposite directions.
19. The surgical device according to paragraph 14, wherein the drive coupling assembly includes:
   a recess defined therein, the recess being configured and dimensioned to receive the distal end of the handle assembly; and
   a pair of lumens transverse to a longitudinal axis defined by the surgical device adapter and at least partially extending through the recess.
20. The surgical device adapter according to paragraph 19, wherein the at least one button includes a pair of posts disposed within the lumens and each of the posts includes a depression configured and dimensioned to release the distal end of the handle assembly in the unclamped configuration.

## Claims

1. A surgical device adapter for coupling an end effector to a handle assembly, the surgical device adapter comprising:
a housing including a proximal end couplable to a distal end of a handle assembly; and
a drive coupling assembly adapted to be selectively couplable to a handle assembly, the drive coupling assembly including a lock assembly having a pair of spring-loaded, opposing buttons movable from a clamped configuration in which the pair of opposing buttons are engaged to a distal end of a handle assembly to an unclamped configuration in which the pair of opposing buttons are disengaged from a distal end of a handle assembly.

2. The surgical device adapter according to claim 1, wherein each of the buttons includes a tab projecting toward one another, whereby each tab is configured and dimensioned to interface with a circumferential slot disposed about a distal end of a handle assembly; preferably wherein each of the buttons includes an actuation portion disposed at an opposite end of the tab.

3. The surgical device adapter according to claim 1 or claim 2, wherein each of the buttons is movable in opposing directions between the clamped configuration and the unclamped configuration; and/or wherein the buttons are biased in opposite directions away from one another.

4. A surgical device adapter for coupling an end effector to a handle assembly, the surgical device adapter comprising:
a housing including a proximal end couplable to a distal end of a handle assembly; and
a drive coupling assembly adapted to be selectively couplable to a handle assembly, the drive coupling assembly including a lock assembly having at least one spring-loaded button movable from a clamped configuration in which the at least one button is engaged to a distal end of a handle assembly to an unclamped configuration in which the at least one button is disengaged from a distal end of a handle assembly.

5. The surgical device adapter according to claim 4, wherein the at least one button includes a tab configured and dimensioned to interface with a circumferential slot disposed about a distal end of a handle assembly, preferably further comprising an opposing, spring-loaded button movable from a clamped configuration in which the buttons are engaged to the distal end of a handle assembly to an unclamped configuration in which the buttons are disengaged from a distal end of a handle assembly.

6. The surgical device adapter according to claim 5, wherein each of the buttons is movable in opposing directions between the clamped configuration and the unclamped configuration; and/or wherein the buttons are biased in opposite directions.

7. The surgical device adapter according to any of claims 4 to 6, wherein the drive coupling assembly includes:
a recess defined therein, the recess being configured and dimensioned to receive a distal end of a handle assembly; and
a pair of lumens transverse to a longitudinal axis defined by the surgical device adapter and at least partially extending through the recess.

8. The surgical device adapter according to claim 7, wherein the at least one button includes a pair of posts disposed within the lumens.

9. The surgical device adapter according to claim 8, wherein each of the posts includes a depression configured and dimensioned to release a distal end of a handle assembly in the unclamped configuration.

10. A surgical device comprising:
an end effector comprising a first jaw and a second jaw moveable relative to the first jaw;
a handle assembly; and
an adapter assembly removably coupled to a proximal end of the end effector and a distal end of the handle assembly, the adapter assembly comprising:
a housing including a proximal end couplable to the distal end of the handle assembly; and
a drive coupling assembly selectively couplable to the handle assembly, the drive coupling assembly including a lock assembly having at least one spring-loaded button movable from a clamped configuration in which the at least one button is engaged to the distal end of the handle assembly to an unclamped configuration in which the at least one button is disengaged from the distal end of the handle assembly.

11. The surgical device according to claim 10, wherein the at least one button includes a tab configured and dimensioned to interface with a circumferential slot disposed about the distal end of the handle assembly.

12. The surgical device according to claim 10 or claim 11, wherein the adapter assembly further comprises an opposing, spring-loaded button movable from a clamped configuration in which the buttons are engaged to the distal end of the handle assembly to an unclamped configuration in which the buttons are disengaged from the distal end of the handle assembly.

13. The surgical device according to claim 12, wherein each of the buttons is movable in opposing directions between the clamped configuration and the unclamped configuration; and/or wherein the buttons are biased in opposite directions.

14. The surgical device according to any of claims 10 to 13, wherein the drive coupling assembly includes:
a recess defined therein, the recess being configured and dimensioned to receive the distal end of the handle assembly; and
a pair of lumens transverse to a longitudinal axis defined by the surgical device adapter and at least partially extending through the recess.

15. The surgical device adapter according to claim 14, wherein the at least one button includes a pair of posts disposed within the lumens and each of the posts includes a depression configured and dimensioned to release the distal end of the handle assembly in the unclamped configuration.
